# EUROPÄISCHE PATENTSCHRIFT

(11) **EP 2 848 679 B1**
(45) Veröffentlichungstag und Bekanntmachung des Hinweises auf die Patenterteilung: **30.12.2015**
(21) Anmeldenummer: 14001771.6
(22) Anmeldetag: 20.05.2014
(51) Int. Cl.: C12M 1/00, B01L 1/02

(54) **Laboratoriumsbrutschrank mit Feuchtigkeitseinstellung**
Laboratory incubator with humidity adjustment
Incubateur avec réglage de l'humidité

(30) Priorität: 16.08.2013 DE 102013013665
(43) Veröffentlichungstag der Anmeldung: 18.03.2015
(73) Patentinhaber: Thermo Electron LED GmbH, 63505 Langenselbold (DE)
(72) Erfinder: Pieczarek, Waldemar, 63505 Langenselbold (DE); Schneider, Jürgen Andreas, 63517 Rodenbach (DE); Stahl, Hermann, 61130 Nidderau-Ostheim (DE)
(74) Vertreter: Tomerius, Isabel

(56) Entgegenhaltungen:
- EP-A1- 1 920 891
- EP-A1- 2 573 162
- EP-A2- 1 552 888
- US-B2- 6 878 177

## Beschreibung

Die Erfindung betrifft einen Laboratoriumsbrutschrank, auch als Inkubator bezeichnet, insbesondere einen Begasungsbrutschrank, mit einem Außengehäuse und einem Innengehäuse, das einen Innenraum begrenzt, in dem Proben gelagert werden können. Der Zugriff auf den Innenraum wird durch eine Tür im Außengehäuse ermöglicht. Gegebenenfalls kann das Innengehäuse eine zusätzliche Innentür aufweisen, die benachbart zur Außentür den Innenraum gesondert verschließt. Diese Innentür kann beispielsweise aus Glas bestehen (vgl. US 6,878,177 B2).

Brutschränke dienen in Laboratorien üblicherweise dazu, in ihrem Innenraum Proben, insbesondere biologische und/oder mikrobiologische Proben, unter vorgegebenen Bedingungen wie einer bestimmten Temperatur und Luftfeuchtigkeit und - im Falle von Begasungsbrutschränken - einer definierten Gasatmosphäre zu lagern. Meist wird dabei versucht, die Bedingungen des menschlichen oder tierischen Körpers zu imitieren. Oft gewählte Bedingungen sind daher eine Temperatur von ca. 37 °C und eine möglichst hohe Luftfeuchtigkeit, die in der Regel bei mindestens 60 %, bevorzugt bei mindestens 80 %, besonders bevorzugt bei mindestens 90 %, liegen sollte, ohne dass dabei jedoch an den Wänden oder anderen Bereichen des Brutschrankes Feuchtigkeit auskondensiert. Derartige Feuchtigkeitsbereiche sind potentielle Kontaminationsherde.

Im Stand der Technik sind verschiedene Möglichkeiten bekannt, in einem Laboratoriumsbrutschrank eine feuchte Innenraumatmosphäre zu erzeugen. Eine erste Möglichkeit besteht darin, im Inneren des Brutschrankes ein Wasserreservoir vorzusehen, aus dem durch Beheizen Wasser verdampft wird (zum Beispiel EP 1552888 A2). Ein großes Problem dieser Lösung ist jedoch die leichte Verkeimung des Wasserbades und die Gefahr der Kontamination der im Brutschrank gelagerten Proben.

Um eine gleichmäßige Temperatur- und Feuchtigkeitsverteilung im Innenraum zu gewährleisten wird die Luft im Innenraum üblicherweise mittels eines Gebläses zirkuliert. Die Strömungsführung erfolgt dabei häufig gleichmäßig über den gesamten Innenraum vom Boden in Richtung Decke, wobei die Luft über das auf dem Boden angeordnete Wasserbad geleitet wird, dabei Feuchtigkeit aufnimmt und diese in den Innenraum transportiert. An der Decke ist ein Gebläse befestigt, das die Luft ansaugt und durch einen Strömungskanal zwischen Außen- und Innengehäuse entlang Decke und Seitenwand zurück zum Boden führt (vgl. US 6,878,177 B2 EP 2 573 162 A1, und US 6,333,004 B1). Eine derartige Ventilation im Innenraum birgt jedoch die Gefahr, dass die dort gelagerten Proben austrocknen oder durch aus dem Wasserbad mitgeschleppte Keime kontaminiert werden. Außerdem wird das Problem beobachtet, dass es oft relativ lange dauert, bis sich die gewünschte Feuchtigkeit und Temperatur im Innenraum beim Starten des Brutschrankes einstellen oder wieder einstellen, wenn die Tür geöffnet und dadurch die Innenraumatmosphäre gestört wurde.

Es bestand also ein Bedarf an einem Laboratoriumsbrutschrank, in dessen Innenraum Proben bei möglichst gleichmäßiger Feuchtigkeit und möglichst geringer Gefahr der Kontamination oder Austrocknung gelagert werden können. Aufgabe der Erfindung ist es, einen solchen Laboratoriumsbrutschrank anzugeben.

Die Lösung dieser Aufgabe gelingt mit dem Laboratoriumsbrutschrank gemäß Anspruch 1. Bevorzugte Weiterbildungen des Laboratoriumsbrutschranks sind in den zugehörigen Unteransprüchen beschrieben.

In ihrem breitesten Aspekt betrifft die Erfindung also einen Laboratoriumsbrutschrank, vorzugsweise einen Begasungsbrutschrank, mit einem eine Tür umfassenden Außengehäuse und einem einen Innenraum umgebenden Innengehäuse, das einen Boden, eine Decke, drei Seitenwände und eine Seitenfläche aufweist, die mit der Tür oder einer zusätzlichen Innentür verschließbar ist, und in welchem ein Strömungskanal vorhanden ist, der wenigstens eine Lufteinströmöffnung in einem Endbereich und wenigstens eine Luftausströmöffnung in einem anderen Endbereich aufweist, wobei die wenigstens eine Luftausströmöffnung so ausgebildet ist, dass ausströmende Luft am Rand des Innenraumes entlang wenigstens einer der Seitenwände und/oder der Seitenfläche geführt wird, und worin innerhalb des Strömungskanals ein beheizbares Wasserreservoir derart angeordnet ist, dass im Strömungskanal fließende Luft über oder durch das Wasserreservoir geleitet wird.

Die verbesserten Eigenschaften des erfindungsgemäßen Laboratoriumsbrutschrankes werden einerseits durch eine spezielle Strömungsführung innerhalb des Innenraumes erreicht sowie andererseits durch die Anordnung des Wasserreservoirs innerhalb des Strömungskanals in der Weise, dass die im Laboratoriumsbrutschrank geführte Luft über oder durch das Wasserreservoir geleitet wird. Die Anordnung des Wasserreservoirs im Strömungskanal führt zu einer räumlichen Trennung desselben vom Innenraum und den dort gelagerten Proben. Gegenüber den in herkömmlicher Weise offen am Boden des Innenraums platzierten Wasserwannen hat dies den Vorteil, dass einerseits die Kontamination des Wasserreservoirs reduziert werden kann und andererseits weniger Keime aus dem Wasser durch die im Innenraum zirkulierende Luft mitgeschleppt werden und die Proben verunreinigen können.

Auch die Zirkulation der Luft im Laboratoriumsbrutschrank, nachfolgend vereinfacht als Inkubator bezeichnet, erfolgt so, dass die Luft innerhalb des Strömungskanals zwischen der wenigstens einen Lufteinströmöffnung und der wenigstens einen Luftausströmöffnung vom Innenraum räumlich abgetrennt erfolgt. Unter einem Strömungskanal im Sinne der Erfindung ist also ein abgeschlossener Raum zu verstehen, in dem Luft durch die wenigstens eine Lufteinströmöffnung eintreten und erst aus der wenigstens einen Luftausströmöffnung in einem anderen Endbereich des Strömungskanals wieder austreten kann. Dies schließt natürlich nicht aus, dass in dem Kanalabschnitt zwischen Lufteinström- und Luftausströmöffnung weitere Zu- oder Auslässe vorhanden sein können. Diese sind jedoch für die im Strömungskanal während des normalen Betriebs des Inkubators geführte Luft nicht passierbar. Beispielsweise können Einmündungen in den Strömungskanal vorhanden sein, durch die weitere Gase wie z.B. Stickstoff oder Kohlendioxid in den Strömungskanal eingeleitet werden. Denkbar sind auch Zuleitungen für Desinfektionsmittel oder ähnliches, gegebenenfalls kombiniert mit einer beispielsweise durch ein Ventil verschlossenen Ablassöffnung, durch die zum Beispiel in einem Reinigungsschritt Reinigungsfluid aus dem Strömungskanal entfernt werden kann.

Wie schon erwähnt, wird während des normalen Betriebs des Inkubators, bei dem im Innenraum Proben unter bestimmten Bedingungen aufbewahrt werden, Luft innerhalb des Strömungskanals ausschließlich derart geführt, dass sie durch die wenigstens eine Lufteinströmöffnung in einem Endbereich des Strömungskanals in diesen eintritt, durch den Strömungskanal geführt wird und ausschließlich durch die wenigstens eine Luftausströmöffnung in einem anderen Endbereich des Strömungskanals aus diesem wieder herausgeführt wird. Dies geschieht in der Weise, dass die ausströmende Luft in einem Randbereich des Innenraums in diesen eintritt und zwar derart, dass die Luft entlang wenigstens einer der Seitenwände und/oder der offenen Seitenfläche, die den Zugang zum Innenraum ermöglicht, geführt wird. Das bedeutet nicht zwangsläufig, dass die Luft unmittelbar auf eine Seitenwand oder die Seitenfläche ausgeblasen werden muss. Vielmehr ist es möglich, die Luft zunächst eine Strecke entlang Decke oder Boden zu führen, sodass sie erst beim Auftreten auf die nächste Seitenwand/Seitenfläche umgelenkt wird und an dieser entlang weiterströmt. Wesentlich ist, dass die Luft nur in einem Randbereich dem Innenraum zugeführt wird, dessen Breite beispielsweise bei maximal 20 %, bevorzugt maximal 15 %, der Breite des Innenraums zwischen der Seitenwand/Seitenfläche, an der die Luft entlang geführt wird, und der gegenüberliegenden Seitenwand/Seitenfläche beträgt. Um die Luftzufuhr aus der Luftausströmöffnung auf den Randbereich zu beschränken, erfolgt die Strömungsführung zweckmäßig parallel oder im Wesentlichen parallel zu der Seitenwand oder Seitenfläche, an der die Luft entlang geführt wird. "Im Wesentlichen parallel" bedeutet dabei, dass der Winkel, mit dem die Luftströmung in Bezug auf die Seitenwand/Seitenfläche geführt wird, höchstens 5 °, insbesondere höchstens 2 °, beträgt. Die ausströmende Luft wird also nicht, wie bisher üblich, über den gesamten Innenraum und damit auch über die Stellflächen für die Proben geführt, sondern ausschließlich in unmittelbarer Nachbarschaft zum Innengehäuse, so dass der Innenbereich des Innenraumes und die dort gelagerten Proben von der ausströmenden Luft nicht unmittelbar getroffen werden. Dies verringert die Gefahr, dass die Proben austrocknen, erheblich.

Die Strömungsführung aus der wenigstens einen Luftausströmöffnung heraus erfolgt bevorzugt so, dass eine im Wesentlichen laminare Strömung erzeugt wird, die möglichst geringe Turbulenzen aufweist. Beispielsweise kann die Luftausströmöffnung in Form einer Schlitzdüse ausgebildet sein, so dass die austretende Luft in Form eines Luftvorhangs am Innengehäuse entlang strömt. Ein Luftaustausch mit dem mittigen Bereich des Innenraums erfolgt also nicht durch direktes Einleiten der ausströmenden Luft in die Innenraummitte, sondern durch die geringen Turbulenzen, die sich am Rande des Luftvorhangs zur Innenraummitte hin ausbilden. Die Strömungsgeschwindigkeiten sind hier jedoch wesentlich geringer als in der laminaren Hauptströmung selbst. Die im Innenraum befindlichen Proben werden durch diese geringen Strömungsgeschwindigkeiten also wenig beeinflusst und trocknen auch erheblich weniger aus als in einer direkten Luftströmung.

Bevorzugt erfolgt die Strömungsführung so, dass sich die wenigstens eine Luftausströmöffnung in einem Randbereich der Decke befindet, die Luft also von oben nach unten im Innenraum strömt. Alternativ oder zusätzlich kann die Luftausströmöffnung in einem der Decke benachbarten oberen Randbereich wenigstens einer der Seitenwände angeordnet sein. Um eine möglichst vertikale Luftströmung von der Decke in Richtung auf den Boden des Innengehäuses hin zu erreichen, ist die wenigstens eine Lufteinströmöffnung, durch die Luft in den Strömungskanal gesaugt wird, bevorzugt in einem Außenrandbereich des Bodens angeordnet und zwar zweckmäßig unterhalb der wenigstens einen Luftausströmöffnung. Grundsätzlich ist es jedoch ebenfalls möglich, die Luftführung umzukehren, d. h. die Anordnung von Lufteinström- und Luftausströmöffnung zu vertauschen, so dass die Luft vom Boden in Richtung auf die Decke geführt wird. In allen Fällen ist es grundsätzlich möglich, Luft entlang nur einer Seitenwand oder der offenen Seitenfläche zu führen oder entlang mehrerer Seitenwände oder einer Seitenwand oder mehreren Seitenwänden und der offenen Seitenfläche.

In einer besonders bevorzugten Ausführungsform erfolgt die Luftströmung ausschließlich entlang der mit einer Tür und gegebenenfalls einer zusätzlichen Innentür verschließbaren offenen Seitenfläche des Innenbehälters des Inkubators. Auch hier ist es bevorzugt, die Luft von oben nach unten zu führen, so dass sich die Luftausströmöffnung in einem Bereich oberhalb der Tür oder der Innentür befindet und die wenigstens eine Lufteinströmöffnung in einem der Unterkante der Tür oder Innentür benachbarten Bereich angeordnet ist. Bei dieser Strömungsführung trifft die feuchte Luft zunächst auf den oberen Bereich der Tür, der etwas wärmer ist als der dem Boden zugewandte Türabschnitt. Dadurch lässt sich eine unerwünschte Kondensation an der Tür besonders gut verhindern. Auch hier ist allerdings wiederum die umgekehrte Strömungsführung von unten nach oben grundsätzlich denkbar.

Ein weiterer Vorteil der Führung der Luftströmung entlang ausschließlich des äußeren Randbereiches des Innenraumes und nicht entlang dessen Mitte besteht darin, dass die Luft an Flächen entlang geführt wird, die üblicherweise beim Betrieb des Inkubators beheizt werden. So besitzen Inkubatoren in der Regel Wandheizungen zur Beheizung der Wände des Innenbehälters und damit des Innenraums. Außerdem ist es üblich, zusätzlich die Tür zu beheizen. Die Beheizung der Flächen, an denen die im Innenraum zirkulierte Luft geführt wird, vermindert das Risiko, dass mit der Luft transportierte Feuchtigkeit an diesen Flächen auskondensiert. Damit verringert sich jedoch das Risiko einer Kontamination des Innenraumes erheblich, da die Gefahr der Bildung von Feuchtepunkten gegenüber herkömmlichen Inkubatoren drastisch reduziert wird.

Der Strömungskanal, durch den die Luft räumlich getrennt vom Innenraum geführt wird, befindet sich aus Platzgründen zweckmäßig außerhalb des Innenraums und insbesondere im Raum zwischen Innen- und Außengehäuse des Brutschranks. Bevorzugt ist dabei eine Anordnung, bei der die Luftströmung insgesamt walzenartig um den Innenraum geführt wird, wobei der Strömungskanal außen entlang des Bodens, einer Seitenfläche und der Decke des Innengehäuses angeordnet ist. Das Wasserreservoir innerhalb des Strömungskanals kann sich dabei prinzipiell an jeder beliebigen Stelle des Kanals befinden. Zweckmäßig ist es jedoch, das Wasserreservoir unterhalb des Bodens des Innengehäuses anzuordnen. Das Wasserreservoir kann als, abgesehen von den Zu- und Ableitungen, geschlossener Behälter ausgebildet sein. Wird die Luft durch das im Wasserreservoir vorhandene Wasser geleitet, ist am Einlass eine Absperrung vorhanden, die verhindert, dass Wasser aus dem Reservoir ausläuft, beispielsweise eine luftdurchlässige, aber wasserundurchlässige Membran, ein Rückschlagventil oder Ähnliches. Bevorzugt ist es, wenn das Wasserreservoir grundsätzlich ähnlich wie die innerhalb des Innenraumes bei den Inkubatoren des Standes der Technik verwendeten Wasserwannen ausgebildet ist. Erfindungsgemäß ist es jedoch hier nicht offen im Innenraum angeordnet, sondern von diesem räumlich getrennt im Strömungskanal. Das Wasser im Wasserreservoir wird auf im Stand der Technik übliche Weise beheizt, beispielsweise durch unterhalb des Bodens der Wasserwanne angeordnete Heizspiralen oder ähnliches. Der gebildete Wasserdampf wird durch die in den Strömungskanal und über oder durch das Wasserreservoir strömende Luft aufgenommen, diese reichert sich mit Feuchtigkeit an und transportiert sie durch den Strömungskanal weiter bis zur wenigstens einen Luftausströmöffnung und von dort in den Innenraum.

Zur Einhaltung der gewünschten Wassertemperatur ist das Wasserreservoir zweckmäßig mit einem Temperatursensor ausgestattet, dessen Messergebnisse in an sich bekannter Weise an eine Auswerte- und Steuereinheit übermittelt werden, die zur Regelung der Heizvorrichtung für das Wasserreservoir eingesetzt wird. Besonders zweckmäßig ist es, den Temperatursensor an einer Stelle des Wasserreservoirs anzuordnen, die der Höhe des minimal erwünschten Wasserspiegels entspricht. Fällt der Wasserstand unter diesen minimalen Wasserspiegel ab, ändern sich die vom Temperatursensor übermittelten Messwerte drastisch. Diese schlagartige Änderung kann als Anzeichen dafür dienen, dass der Wasserstand im Wasserreservoir erhöht werden muss. Die Auswerte- und Steuereinheit kann entsprechend einen Warnhinweis an den Benutzer ausgeben oder automatisch dafür sorgen, dass Wasser in das Wasserreservoir nachgefüllt wird. Die daraufhin erforderliche Neueinstellung der Wassertemperatur, die Auswirkungen auf Temperatur und Feuchtigkeit im Innenraum hat, ist wegen der erfassbaren Temperaturmesswerte schneller und genauer möglich als bisher, wo erst dann ein Nachheizen des Wasserreservoirs erfolgt, wenn sich Temperatur- und/oder Feuchtigkeitsänderungen im Innenraum bemerkbar machen.

Im Stand der Technik ist es bisher üblich, die Temperatur des Innenraumes des Inkubators und die Temperatur des Wasserbades gemeinsam zu regeln. Dies ist im Rahmen dieser Erfindung ebenfalls möglich. Bevorzugt ist jedoch eine getrennte Regelung der Heizvorrichtung für den Innenraum und der Heizvorrichtung für das Wasserreservoir. Die Temperaturregelung entsprechend den Messergebnissen jeweils getrennter Temperatursensoren für den Innenraum und das Wasserreservoir hat den Vorteil, dass eine wesentlich genauere Temperatureinstellung bei gleichzeitig besseren Erholzeiten nach Störung des Systems möglich wird. Eine solche Störung kann beispielsweise durch Änderungen der Außentemperatur oder durch Öffnen der Tür zum Innenraum erzeugt werden. Um den dadurch verursachten und für die Proben nachteiligen Feuchtigkeits- und Temperaturverlust auszugleichen, ist es sinnvoll, die Heizvorrichtung für das Wasserbad stärker zu betreiben und das Heizverhältnis von Innenwand-Heizung zu Wasserreservoir-Heizung zu Gunsten letzterer zu verändern. Bei einer Kopplung der beiden Heizvorrichtungen ist dies natürlich nicht möglich. Nach dem Schließen der Tür darf jedoch die Heizleistung für das Wasserreservoir nicht allzu lange erhöht bleiben, da es sonst zu einem unbeabsichtigt hohen Feuchtigkeitseintrag in den Innenraum und dann gegebenenfalls zu einem Auskondensieren der Feuchtigkeit dort kommt. Auch diese spezifische Regelung ist in der erfindungsgemäßen Vorrichtung erheblich einfacher möglich, da die Entkopplung von Innenraum-Heizung und Wasserreservoir-Heizung eine sehr viel genauere und zeitverkürzte Einstellung der gewünschten Temperatur- und Feuchtigkeitswerte erlaubt.

Eine noch genauere Regelung der Feuchtigkeit im Innenraum ist durch die erfindungsgemäße Auslegung des Kältepunktes möglich. Ein Kältepunkt, auch als "Cold spot" bezeichnet, ist ein Bereich mit einer gegenüber der Innenraumtemperatur verringerten Temperatur, der zum Auskondensieren übermäßiger Feuchtigkeit aus dem Innenraum eingesetzt wird. Bei den Inkubatoren des Standes der Technik befindet sich ein solcher Kältepunkt üblicherweise im Innenraum selbst und ist als Teil der Innenraumwandung ausgelegt, in dem die Temperatur durch Anlegen einer Kältebrücke nach außen gegenüber den umliegenden Gehäuseteilen reduziert wird. Meist befindet sich dieser Kältepunkt in einem unteren Seitenwandbereich des Innengehäuses. Übermäßig im Innenraum vorhandene Feuchtigkeit kondensiert an dieser Stelle aus. Im Innenraum ist daher immer ein Feuchtigkeitspunkt vorhanden, der eine Quelle nicht erwünschter Kontamination darstellt. Erfindungsgemäß wird dieser Kältepunkt nun aus dem Innenraum heraus verlegt und zwar in den Strömungskanal, in Strömungsrichtung dem Wasserreservoir nachgeordnet. Dieser Kältepunkt erfüllt denselben Zweck wie im Stand der Technik, nämlich dort überschüssige Feuchtigkeit auszukondensieren. Durch die räumliche Trennung des Wasserreservoirs und des nachgeschalteten Kältepunktes ist jedoch die Gefahr von Kontamination im Innenraum gegenüber den Lösungen des Standes der Technik deutlich reduziert.

Prinzipiell kann der Kältepunkt wie im Stand der Technik ausgebildet sein. Es ist möglich, ihn passiv oder aktiv zu kühlen. In der einfachsten Variante kann es beispielsweise ausreichend sein, den Kältepunkt an eine Stelle zu legen oder mit einer Stelle wärmeleitend zu kontaktieren, deren Temperatur ausreichend niedrig ist. Zudem kann ein Temperiermittel zur Einstellung der Temperatur des Kältepunktes vorgesehen sein. Dabei kann es sich beispielsweise um eine Temperaturbrücke handeln, die mit dem Außengehäuse wärmeleitend kontaktiert ist. In üblicher Weise ist dies zum Beispiel ein Metall- und insbesondere Aluminiumstreifen. Alternativ kann zur Kühlung ein Hohlkörper verwendet werden, der mit einem Kühlfluid gefüllt ist. Dies kann konkret eine Rohrleitung sein, durch die Wasser oder ein anderes Kühlfluid geleitet wird. In einer bevorzugten Variante kann das Kühlfluid aus dem Hohlkörper entleert werden. Dies hat den Vorteil, dass der Kältepunkt durch Entleerung des Kühlfluids praktisch abgeschaltet werden kann. Ist das Kühlfluid im Hohlkörper zur Wärmeleitung nicht mehr vorhanden, wird auch der Kältepunkt nicht mehr gekühlt, und seine Temperatur passt sich im Wesentlichen der Umgebungstemperatur an. Von Nutzen kann dies sein, wenn die Temperatur im Innenraum beispielsweise für ein Hochtemperatur-Desinfektionsverfahren insgesamt erhöht werden soll. Eine wärmeableitende Kältebrücke aus dem Innenraum heraus wäre in diesem Fall kontraproduktiv und würde die Erwärmung des Innenraumes verzögern. Nach Abschluss eines solchen Desinfektionsverfahrens kann der Hohlkörper dann wieder mit Kühlfluid gefüllt werden, so dass er seine Funktion als Kältebrücke wieder aufnehmen kann. Das Befüllen kann dabei entweder von Hand oder automatisch, beispielsweise mit einer geeigneten Pumpvorrichtung, die an ein Kühlfluidreservoir angeschlossen ist, erfolgen.

In einer weiteren Ausführungsform kann die Temperierung des Kältepunktes aktiv erfolgen, beispielsweise mit einer Temperiervorrichtung in Form eines Gebläses oder einer elektrischen Kühl- und/oder Heizvorrichtung. Letztere ermöglicht die rasche Erwärmung des Kältepunktes in bestimmten Anwendungssituationen, in denen beispielsweise ein Auskondensieren von Feuchtigkeit vermieden werden soll. Dies kann ein schon erwähnter Desinfektionsvorgang sein oder eine Störungssituation, bei der ein unerwünschter Abfall der Luftfeuchtigkeit möglichst rasch ausgeglichen werden soll.

Um eine gezielte Temperierung des Kältepunktes zu erreichen, ist am Kältepunkt bevorzugt ein Temperatursensor vorgesehen. Anhand der von diesem gelieferten Messergebnisse kann dann die Temperiervorrichtung entsprechend der gewünschten Temperatur des Kältepunktes geregelt werden. Dabei ist es möglich, die Temperatur des Kältepunktes in Abhängigkeit von den im Innenraum gewünschten Bedingungen gezielt vorab festzulegen. Eine höhere Temperaturvorgabe für den Kältepunkt resultiert in einer höheren Luftfeuchtigkeit im Innenraum. Dies kann zum Beispiel sinnvoll sein, wenn Mikrotiterplatten im Innenraum aufbewahrt werden sollen. Diese weisen pro Probe ein sehr geringes Volumen auf, was die Gefahr der Austrocknung erhöht. Umgekehrt kann es sinnvoll sein, für Proben mit einem hohen Flüssigkeitsgehalt und hoher Verdunstungsrate die Luftfeuchtigkeit im Innenraum relativ niedrig zu halten, um ein unerwünschtes Auskondensieren von Feuchtigkeit im Innenraum zu vermeiden. In derartigen Fällen kann eine geringere Temperatur für den Kältepunkt vorgegeben werden. Prinzipiell ist es dabei möglich, den Parameter "Temperatur des Kältepunktes" von Anfang an auf einen bestimmten Wert festzulegen. Dies kann auch in Absprache mit dem Benutzer des Inkubators und abhängig von der beabsichtigten Verwendung vorab geschehen. Alternativ können dem Benutzer Wahlmöglichkeiten für diesen Parameter bereitgestellt werden, aus denen er von Anwendung zu Anwendung auswählen kann. In ähnlicher Weise kann auch der Parameter "Temperatur des Wasserreservoirs" vorab auf einen geeigneten Wert festgelegt werden, oder es können verschiedene Parameterwerte zur Auswahl während des Betriebs vorgesehen sein.

Die Möglichkeit der gezielten Temperierung des Kältepunktes bei gleichzeitig spezifisch regelbarer Temperatur des Wasserreservoirs erlaubt eine bisher nicht möglich gewesene gezielte Einstellung der Feuchtigkeit im Innenraum. Nicht nur können die gewünschten Feuchtigkeitswerte sehr exakt eingehalten werden, sondern es ist auch möglich, diese sehr schnell auf die gewünschten Werte zu bringen und auf festgestellte Abweichungen in minimaler Zeit zu reagieren. Die bereits angesprochene sehr schnelle Rückstellung der Feuchtigkeitswerte im Innenraum bei einer Störung des Systems, wie sie beispielsweise durch das Öffnen der Tür ausgelöst werden kann, lässt sich durch die unabhängige Temperierung des Kältepunktes weiter verbessern. Bei einer Reduktion der Feuchtigkeit im Innenraum durch das Öffnen der Tür ist es beispielsweise möglich, die Temperatur des Kältepunktes zu erhöhen und so eine Auskondensation von Wasserdampf am Kältepunkt zu verhindern. Damit wird, bei der gleichzeitigen Erhöhung der Temperatur des Wasserreservoirs, wie vorstehend beschrieben, sehr schnell sehr viel Feuchtigkeit in den Innenraum transportiert, so dass sich dort die Luftfeuchtigkeit rasch wieder erholt. Andererseits kann nach dem Schließen der Tür durch Absenken der Temperatur des Kältepunktes verhindert werden, dass aus dem noch sehr warmen Wasserreservoir unerwünscht Feuchtigkeit in den Innenraum vordringt und dort auskondensiert, indem die überflüssige Feuchtigkeit bereits an dem abgekühlten Kältepunkt aus der feuchten Luftströmung entfernt wird. Ein Überschießen der Feuchtigkeit im Innenraum kann daher auf besonders sichere Weise verhindert werden.

Wie schon im Falle der Heizvorrichtung für das Wasserreservoir ist es auch im Fall der Temperiervorrichtung für den Kältepunkt bevorzugt, diese in Abhängigkeit von Messergebnissen zu regeln, die bestimmte Betriebsparameter des Inkubators betreffen. Insbesondere werden bei der Regelung der Temperiervorrichtung für den Kältepunkt und/oder der Heizvorrichtung des Wasserreservoirs Messergebnisse wenigstens eines der folgenden Sensoren berücksichtigt: einem Sensor zur Bestimmung der Temperatur im Innenraum, einem Sensor zur Bestimmung der Feuchtigkeit im Innenraum, einem Sensor zur Bestimmung, ob die Tür geöffnet oder geschlossen ist, einem Sensor zur Bestimmung, ob die Innentür geöffnet oder geschlossen ist.

Durch die kombinierte Regelung von Heizvorrichtung für das Wasserreservoir und Temperiervorrichtung für den Kältepunkt können vor allem die Feuchtigkeitswerte im Innenraum sehr gezielt und in kurzer Zeit auf die gewünschten Feuchtigkeitswerte eingestellt werden. Dabei ist es aufgrund der Separierung des Wasserreservoirs und des Kältepunktes möglich, größere Temperaturdifferenzen zwischen beiden vorzugeben, als dies im Stand der Technik bislang möglich war. Während bisher maximale Temperaturdifferenzen von 1 °C üblich waren, sind nun Temperaturdifferenzen von bis zu 3 °C einstellbar. Dadurch wird der Regelbereich für Temperatur und Feuchtigkeit im Innenraum erheblich erweitert. Außerdem ist es über die getrennte Bestimmung der Temperaturen von Wasserreservoir und Kältepunkt sowie deren getrennte Regelung möglich, die Anlaufphase nach einem Neustart oder Erststart des Inkubators gegenüber den herkömmlichen Startphasen zu verkürzen. Die Startphase wird üblicherweise dann als beendet betrachtet, wenn über einen vorgegebenen längeren Zeitraum die Luftfeuchtigkeit im Innenraum konstant bleibt. Die Messung derselben erfolgt üblicherweise durch Nullabgleich einer Wärmeleitfähigkeits-Messzelle. Im Fall des erfindungsgemäßen Inkubators stehen nun weitere Werte, nämlich die Wasserbadtemperatur und die Kältepunkt-Temperatur, zur Verfügung, so dass die Anlaufphase bereits dann als beendet betrachtet werden kann, wenn die Wasserbadtemperatur und die Temperatur des Kältepunktes einen Sollwert erreicht haben. Diese Sollwerte werden besonders schnell dann erreicht, wenn die Temperatur des Wasserbades unabhängig von der Innenraumtemperatur eingestellt und überwacht werden kann. Selbst im Falle des Überschwingens der Temperatur des Wasserreservoirs, wie sie beispielsweise in raschen Aufheizphasen vorkommen kann, führt in der Regel nicht zu einem unerwünschten Anstieg der Luftfeuchte im Innenraum, da durch die gesonderte Regelung des Kältepunktes überschüssige Feuchtigkeit aus der Luft wieder auskondensiert wird, bevor sie den Innenraum erreicht. Anstelle der getrennten Regelung der Temperaturen von Wasserreservoir und Kältepunkt ist in einer einfacheren Variante auch ein Inkubator ohne Regelung realisierbar, bei dem das Heizverhältnis von Wasserreservoir und Kältepunkt fest voreingestellt ist. Einige der beschriebenen Vorteile können dann aber nicht oder nur eingeschränkt erreicht werden.

Die exakte Einstellbarkeit der Luftfeuchtigkeit im Innenraum des erfindungsgemäßen Inkubators verhindert, dass dort unerwünscht Feuchtigkeit auskondensiert und eine Kontaminationsquelle bildet. Eine weitere Kontaminationsquelle stellt zudem das Wasserreservoir dar. Die Gefahr, dass Keime aus dem Wasserbad in den Innenraum gelangen und dort die Proben verunreinigen, ist im erfindungsgemäßen Inkubator bereits dadurch deutlich reduziert, dass das Wasserreservoir im Strömungskanal angeordnet ist und nicht unmittelbar im Innenraum, sondern räumlich von diesem getrennt. Zudem bietet die Anordnung des Wasserbades im Strömungskanal die Möglichkeit, vor und/oder nach dem Wasserreservoir einen Filter anzuordnen. Ein in Strömungsrichtung vor dem Wasserreservoir angeordneter Filter verhindert das Eindringen von Keimen oder sonstigen Verschmutzungen in das Wasserreservoir und verlängert so die Zeit bis zum Austausch des Wassers. Ein in Strömungsrichtung nach dem Wasserreservoir angeordneter Filter verhindert, dass eventuell im Wasser vorhandene Keime oder sonstige Verunreinigungen mit der durch oder über das Wasserreservoir geführten Luft stromabwärts in Richtung auf die wenigstens eine Luftauslassöffnung und von dort heraus in den Innenraum befördert werden. Werden als Filter herkömmliche HEPA- oder ULPA-Filter verwendet, wie sie grundsätzlich bereits in Inkubatoren im Einsatz sind, können die üblicherweise in Inkubatoren auftretenden Keime zurückgehalten werden. Es sind jedoch auch bereits weniger wirksame Filter einsetzbar, beispielsweise in Form von Membranen oder sonstigen üblichen Filtermaterialien, die zwar keine Keimfreiheit garantieren, aber zumindest sonstige Verschmutzungen zurückhalten. Solche weniger wirksamen Filter können beispielsweise am Eingang des Wasserreservoirs eingesetzt werden. Am Ausgang des Wasserreservoirs in Richtung auf den Innenraum des Inkubators hin werden jedoch vorzugsweise HEPA- oder ULPA-Filter verwendet, um das Vordringen von Keimen in den Innenraum zu verhindern.

Zur Förderung der Luft innerhalb des Strömungskanals und im Innenraum des Inkubators wird zweckmäßig ein Gebläse eingesetzt, wie es grundsätzlich bereits zuvor in Läboratoriumsbrutschränken verwendet wurde. Beispielsweise kann ein Radialgebläse eingesetzt werden. Das Gebläse wird zweckmäßig ebenfalls im Strömungskanal angeordnet. Grundsätzlich kann es sich dort an jeder geeigneten Stelle befinden, vorzugsweise wird es jedoch stromabwärts nach dem Wasserreservoir und insbesondere nach dem Kältepunkt angeordnet. Um zu verhindern, dass von dem Gebläse erzeugte Verunreinigungen in den Innenraum transportiert werden, kann stromabwärts des Gebläses im Strömungskanal, insbesondere in Nachbarschaft zu der wenigstens einen Luftausströmöffnung, ein Filter, beispielsweise ebenfalls wieder eine luftdurchlässige Membran, ein HEPA- oder ULPA-Filter, angeordnet sein. Da im Strömungskanal zwischen Gebläse und Filter Überdruck herrscht, sollte der Kanal in diesem Bereich soweit abgedichtet sein, dass Luft nicht am Filter vorbei aus dem Strömungskanal austreten kann.

Besonders zweckmäßig ist es, das Gebläse so zu betreiben, dass im Innenraum und im Strömungskanal eine möglichst konstante Strömungsgeschwindigkeit vorherrscht. Sind im Strömungskanal Filter angeordnet, steigt der Strömungswiderstand mit zunehmender Belegung der Filter allmählich an. Um einen konstanten Volumenstrom sicherzustellen, ist es daher erforderlich, die Gebläseleistung entsprechend anzupassen, konkret heraufzusetzen, wenn die Filter allmählich verstopfen. Dies kann dadurch erreicht werden, dass einem Filter im Strömungskanal ein Strömungswiderstandsmesser zugeordnet ist und die Gebläseleistung in Abhängigkeit von den Messergebnissen dieses Strömungswiderstandsmessers geregelt wird. Treten Störungen des Systems auf, wie durch Öffnen der Tür oder der Türen zum Innenraum, kann eine Änderung der Gebläseleistung sinnvoll sein. Ist die Luftfeuchtigkeit nach Öffnen der Tür abgesunken, kann eine schnellere Wiederherstellung der gewünschten Feuchtigkeitswerte zum Beispiel dadurch erreicht werden, dass nach Schließen der Tür(en) die Gebläseleistung erhöht wird, um mehr Feuchtigkeit in den Innenraum zu fördern. Nach Erreichen des Sollwerts kann die Gebläseleistung dann wieder auf die normale Förderleistung gesenkt werden. Außerdem können kurzzeitig erhöhte Gebläseleistungen nützlich sein, wenn Verunreinigungen in den Inkubator gelangt sind, die durch rasche Förderung über einen HEPA- oder ULPA-Filter möglichst schnell aus dem System wieder entfernt werden sollen. Eine andere Gebläseleistung als im normalen Probenlagerungsbetrieb kann auch für andere Anwendungsschritte, wie beispielsweise bei einer Desinfektion oder beim Start oder Neustart des Inkubators, gewählt werden. Eine raschere Einstellung der Innenraumatmosphäre durch erhöhte Gebläseleistung verringert zum Beispiel die Ansprechzeit der Sensoren und verkürzt so die Totzeit des Systems. Außerdem können Sensoren in größerem Abstand zueinander angeordnet werden, da in kürzerer Zeit an weit voneinander entfernt gelegenen Stellen gleiche Bedingungen eingestellt werden können. Außerdem können aus dem gleichen Grund Gaseinlässe und zugehörige Gassensoren weiter voneinander entfernt angeordnet werden. Liegen umgekehrt Gaseinlass und zugehöriger Sensor zu nahe beieinander, kann die Gebläseleistung gesenkt werden, um die Totzeit zu verlängern. Dieses Vorgehen ist auf andere Sensoren und Messparameter übertragbar.

Im Strömungskanal und im Innenraum des Inkubators kann nicht nur Luft gefördert werden, sondern auch eine andere Gasatmosphäre. Beispielsweise ist es in so genannten Begasungsbrutschränken üblich, die Atmosphäre mit Kohlendioxid anzureichern, um einen sauren pH-Wert einzustellen. Bei dem erfindungsgemäßen Laboratoriumsbrutschrank ist dies ebenfalls möglich. Es besteht sogar ein besonderer Vorteil gegenüber herkömmlichen Brutschränken, bei denen häufig beobachtet, wird, dass die Luftfeuchtigkeit sich durch Einleitung von weiteren Gasen zusätzlich zur Luft in unerwünschter Weise herabsetzt. Erfindungsgemäß kann dies dadurch verhindert werden, dass das Gas, beispielsweise Stickstoff oder Kohlendioxid, in den Strömungskanal eingeleitet wird, und zwar zweckmäßig stromaufwärts des Wasserreservoirs. Bevor das Gas also in den Innenraum des Inkubators gelangt, wird es über oder durch das Wasserbad geführt, reichert sich dort mit Feuchtigkeit an und wird zusammen mit der im Strömungskanal geförderten Luft mit der gewünschten Luftfeuchtigkeit dem Innenraum zugeführt. Ein Austrocknen der Proben wird deshalb wirksam verhindert.

Die Erfindung soll nachfolgend anhand von Zeichnungen näher erläutert werden. Die Zeichnungen sind rein schematisch und dienen lediglich der Beschreibung bevorzugte Ausführungsformen der Erfindung, ohne dass diese auf die beschriebenen Ausführungsformen beschränkt wäre. In den Zeichnungen bezeichnen gleiche Bezugszeichen gleiche Teile. In den Figuren ist
- Fig. 1: eine Draufsicht auf die Vorderseite eines erfindungsgemäßen Laboratoriumsbrutschranks;
- Fig. 2: eine Schnittansicht entlang der Linie A-A der Figur 1 in einer leicht abgewandelten Ausführungsform;
- Fig. 3: eine vereinfachte Ansicht der Figur 2 zur Veranschaulichung der Strömungsführung im Innenraum;
- Fig. 4: eine Ausschnittvergrößerung des Bereichs B der Figur 2 in einer etwas abgeänderten Ausführungsform des Inkubators und
- Fig. 5: eine weitere Ausschnittvergrößerung des Bereichs B der Figur 2 in einer weiteren abgeänderten Variante.

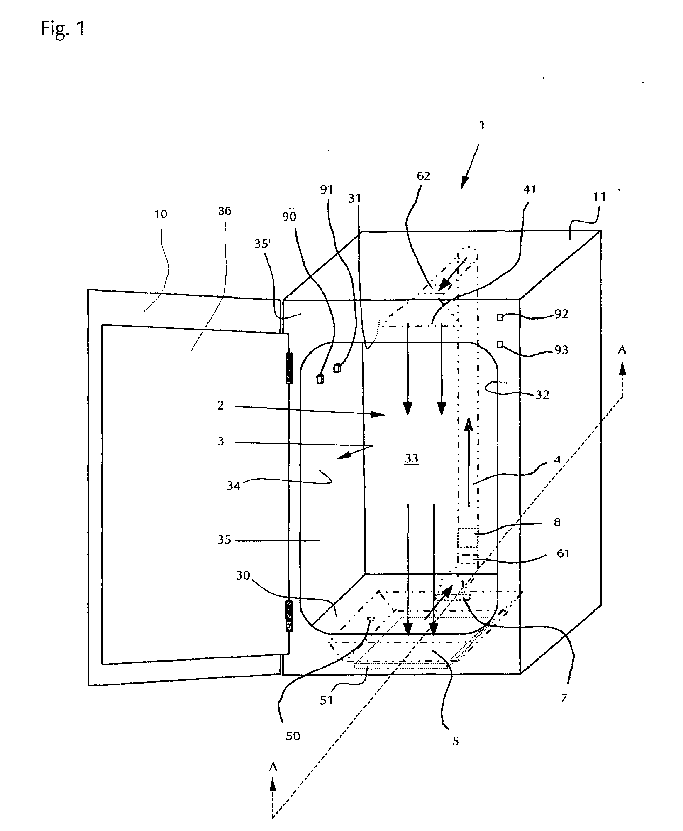

Figur 1 zeigt einen erfindungsgemäßen Laboratoriumsbrutschrank 1, hier am Beispiel eines Begasungs-Inkubators, in einer Draufsicht auf dessen Frontseite. Der Laboratoriumsbrutschrank besitzt ein Außengehäuse 11, das eine Außentür 10 aufweist, die den Zugang zu einem Innenraum 2 ermöglicht. Der Innenraum 2 wird von einem Innengehäuse 3 umschlossen, das aus einem Boden 30, Seitenwänden 32, 33 und 34 sowie einer Decke 31 besteht. Die auf den Benutzer zuweisende Seitenfläche 35 ist, abgesehen von einer Frontblende 35', offen und ermöglicht den Zugriff auf den Innenraum 2. Die Seitenfläche 35 kann mit einer zusätzlichen Innentür 36 verschlossen werden, die beispielsweise aus Glas besteht. Im gezeigten Fall sind beide Türen 10 und 36 geöffnet dargestellt.

Der Innenraum 2 dient zur Aufbewahrung von Proben, beispielsweise mikrobiologischen Proben, die auf im Innenraum angeordneten Tragböden gelagert werden können. Proben und Tragböden sind der Übersichtlichkeit halber hier jedoch weggelassen. Für die Aufbewahrung der Proben wird im Innenraum 2 eine definierte Atmosphäre mit einer vorgegebenen Temperatur, beispielsweise 37 °C, und einer vorgegebenen Luftfeuchtigkeit, vorzugsweise von mindestens 80 % r. F., besser noch mindestens 90 %, eingestellt. Um eine Beschädigung der Proben zu verhindern, müssen diese Bedingungen möglichst konstant gehalten werden und sollten nach einer Störung, wie sie beispielsweise durch das Öffnen der Türen 10 und 36 hervorgerufen werden kann, möglichst rasch wieder hergestellt werden können.

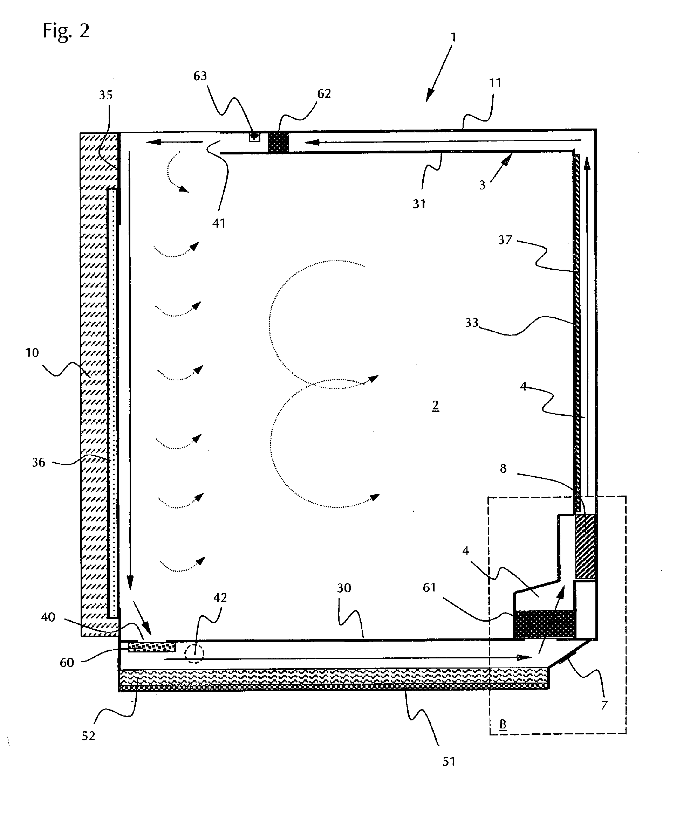

Zur Befeuchtung der Innenraumatmosphäre ist im Inkubator ein Wasserreservoir 5 angeordnet, das während des Betriebs des Inkubators 1 mit Wasser gefüllt ist. Das im Wasserreservoir 5 vorhandene Wasser kann mittels einer am Boden des wannenförmigen Wasserreservoirs 5 angeordneten Heizvorrichtung 51 erwärmt werden. Zur Einstellung der Temperatur im Innenraum 2 ist eine Wandheizvorrichtung 37 angeordnet (vergleiche Figur 2), die die Seitenwände 32, 33 und 34 beheizt. Die Wandheizvorrichtung 37 sowie die Heizvorrichtung 51 für das Wasserreservoir 5 können im gezeigten Beispiel getrennt voneinander betrieben werden.

Wie in Figuren 1 und 2 erkennbar, ist im erfindungsgemäßen Inkubator das Wasserreservoir 5 außerhalb des Innenraums 2 angeordnet, nämlich unterhalb der Bodenplatte 30. Der Boden 30 ist, abgesehen von einer in Fig. 1 durch die Frontblende 35' verdeckten Lufteinströmöffnung, eine zum Innenraum 2 hin geschlossene Fläche. Um Feuchtigkeit aus dem Wasserreservoir 5 in den Innenraum 2 zu transportieren, wird Luft durch den Inkubator zirkuliert. Die Luft tritt durch die Lufteinströmrichtung 40 (siehe Fig. 2) im Boden 30, benachbart zur Frontöffnung des Innengehäuses und der Seitenfläche 35, in einen Strömungskanal 4 ein, der unterhalb der Bodenfläche 30 von der Seitenfläche 35 bis zur rückwärtigen Seitenwand 33 verläuft, dann hinter dieser entlang bis nach oben zur Decke 31 und dann der Decke entlang bis in Richtung auf die Seitenfläche 35 hin, wo der Strömungskanal 4 in einer Luftausströmöffnung 41 endet. Durch diese, in Fig. 1 schlitzdüsenartige, Luftausströmöffnung 41 heraus kann die im Strömungskanal mittels eines Gebläses 8, das hinter der Rückwand 33 des Innengehäuses 3 angeordnet ist, zirkulierte Luft in den Innenraum 2 austreten.

In der Ausführungsform der Fig. 1 ist die schlitzdüsenförmige Luftausströmöffnung dicht benachbart und parallel zur Seitenfläche 35 angeordnet, so dass die Luft aus der Luftausströmöffnung heraus direkt von oben nach unten an der Seitenfläche 35 entlang strömt. Fig. 2 zeigt eine etwas veränderte Anordnung der Luftausströmöffnung 41. Hier endet der Strömungskanal 4 an der Decke 31 in einem Abstand von der benachbarten Seitenfläche 35. Die Luft wird zunächst eine kurze Strecke unmittelbar entlang der Decke 31 geführt. Sie biegt an Seitenfläche 35 in Richtung auf den Boden 30 hin ab und fließt an der Seitenfläche 35 bzw. der diese im Betrieb verschließenden Innentür 36 entlang in einem äußeren Randbereich des Innenraumes 2 nach unten in Richtung auf die Lufteinströmöffnung 40, wo die Luft wieder in den Strömungskanal 4 eintritt. Insgesamt wird die Luft also in den Ausführungsformen der Fig. 1 und 2 walzenartig am Rand des Innenraumes 2 entlang um die Innenraummitte herum geführt, wie dies insbesondere in Figur 2 zu erkennen ist. Der Luftstrom ist in den Figuren 1 und 2 durch die durchgezogenen Pfeile gekennzeichnet. Diese walzenartige Strömungsführung um den zentralen Innenraum herum hat den Vorteil, dass in der Innenraummitte gelagerte Proben nicht unmittelbar von der zirkulierenden Luft getroffen werden. Dadurch sind sie einer geringeren Gefahr von Austrocknung ausgesetzt, als dies in Inkubatoren des Standes der Technik der Fall ist, wo Luft über den gesamten Innenraumbereich meist von unten nach oben oder in umgekehrter Richtung geführt wird und so die Proben unmittelbar trifft. Im erfindungsgemäßen Inkubator dagegen erfolgt ein Luftaustausch mit dem zentralen Innenraumbereich nur durch eine Sekundärströmung, die durch Verwirbelungen am Rand der Hauptströmung entsteht. Dies wird durch die gebogenen und gestrichelten Pfeile in Figur 2 veranschaulicht. Da die Sekundärströmung eine wesentlich geringere Strömungsgeschwindigkeit besitzt als die Hauptströmung, ist die Austrocknung geringer, als wenn die Proben der Hauptströmung ausgesetzt wären.

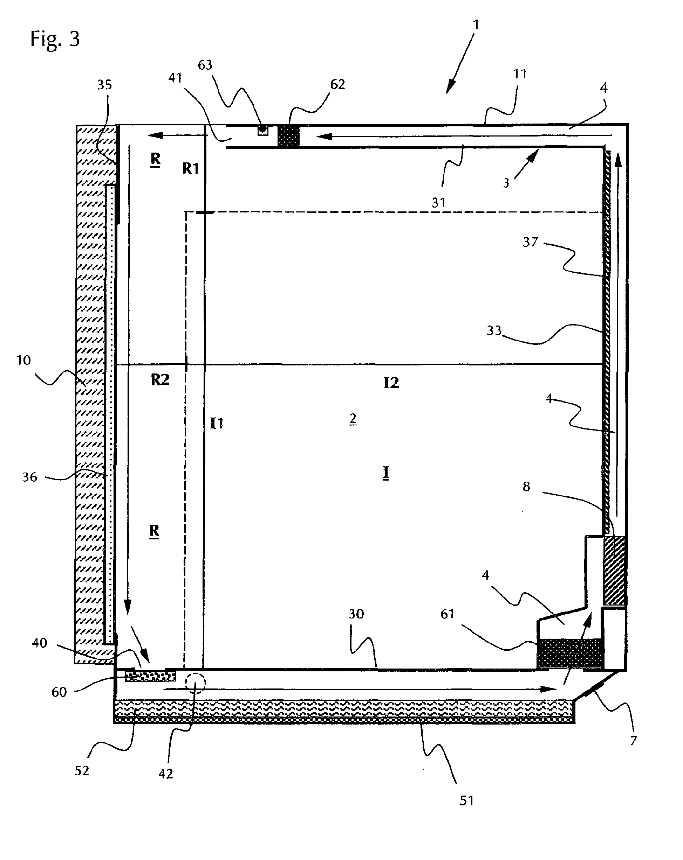

Der Randbereich, in dem die Strömungsführung im Innenraum erfolgt, ist skizzenartig in Fig. 3 dargestellt. Die Figur veranschaulicht die Bereiche des Innenraums, denen unmittelbar Luft zugeführt wird, im Verhältnis zu den Innenraumbereichen, die von der Luftströmung nicht unmittelbar getroffen werden. Derjenige Bereich, dem Luft aus der Luftausströmöffnung unmittelbar zugeführt wird, ist der im linken Teil der Figur mit R bezeichnete Randbereich, benachbart zur Seitenfläche 35 und der oben an die Seitenfläche 35 angrenzenden Decke 31. Der Bereich, der von der aus der Luftausströmöffnung 41 austretenden Luft nicht unmittelbar getroffen wird, ist mit I bezeichnet. Die Breite (R1 bzw. R2) des Randbereichs R plus die Breite (11 bzw. 12) des an den Randbereich angrenzenden Innenraumbereichs entspricht der Gesamtbreite des Innenraumes zwischen der den Innenraum begrenzenden Seitenwand 33 und der Seitenfläche 35 bzw. zwischen Decke 31 und Boden 30. Die Breite des Randbereichs, dem die Luft aus dem Strömungskanal 4 zugeführt wird, beträgt maximal 20 % der Gesamtbreite des Innenraums - also hier R1/(R1 + I1) ≤ 0,2 bzw. R2/(R2 + 12) ≤ 0,2. In den übrigen Innenraumbereich I erfolgt keine unmittelbare Zufuhr von Luft aus dem Strömungskanal. Ein Luftaustausch erfolgt dort aufgrund der von der Hauptströmung ausgehenden Sekundärströmung.

Um die Luftzufuhr aus der Luftausströmöffnung 41 auf den Randbereich R zu beschränken, erfolgt die Strömungsführung zweckmäßig parallel oder im Wesentlichen parallel zu der Seitenwand oder Seitenfläche, an der die Luft entlang geführt wird. "Im Wesentlichen parallel" bedeutet dabei, dass der Winkel der Luftströmung in Bezug auf die Seitenwand/Seitenfläche, an der die Luftströmung entlang fließt, höchstens 5 °, insbesondere höchstens 2 °, beträgt. Im gezeigten Fall wird die Luft parallel zur Deckenfläche, also mit einem Winkel von 0 ° zu dieser, in den Innenraum eingeblasen.

Der Transport von Luftfeuchtigkeit in den Innenraum 2 erfolgt dadurch, dass die im Strömungskanal 4 fließende Luft nach dem Eintritt durch die Lufteinströmöffnung 40 über das Wasserreservoir 5 geleitet wird. Bevorzugt geschieht dies, wie in Figuren 1 und 2 veranschaulicht, in der Weise, dass die Luft einmal über im Wesentlichen die gesamte Breite des Wasserreservoirs 5 hinübergeführt wird, so dass eine lange Wegstrecke zur Verfügung steht, auf der sich die Luft mit Wasserdampf anreichern kann. Um das Einschleppen von Verschmutzungen ins Wasserreservoir zu reduzieren, ist über der Lufteintrittsöffnung 40 ein Filter 60, beispielsweise in Form einer Filtermembran, angeordnet. Am Ausgang des Wasserreservoirs 5 ist im Strömungskanal 4 ein HEPA-Filter 61 angeordnet, den die mit Wasserdampf angereicherte Luft passieren muss, bevor sie durch das Gebläse 8 im Strömungskanal 4 weiter gefördert wird und durch die Luftausströmöffnung 41 hinaus in den Innenraum 2 eintritt. Dadurch wird verhindert, dass eventuell im Wasserreservoir 5 vorhandene Keime von der Luft in den Innenraum mitgeschleppt werden und dort die Proben verunreinigen.

Da das zum Transport der Luft verwendete Gebläse 8 ebenfalls Verunreinigungen freisetzen kann, ist im Strömungskanal 4 vor der Luftausströmöffnung 41 ein weiterer Filter 62 angeordnet, der derartige Verunreinigungen zurückhält. Bei dem Strömungskanal 4 handelt es sich also um ein für die Luftströmung geschlossenes System, das mehrfach mit Filtern gegen Verschmutzung bzw. Verkeimung gesichert ist. Auf diese Weise ist das von der Bodenplatte 30 vom Innenraum 2 getrennte und mit Filtern 60 und 61 eingerahmte Wasserreservoir 5 in besonderer Weise vor Verunreinigungen und Verkeimung geschützt. Weder können Verunreinigungen von Proben, die im Innenraum 2 gelagert sind, unmittelbar das Wasserreservoir verschmutzen, da eine räumliche Trennung durch die Bodenplatte 30 vorhanden ist, noch können umgekehrt Keime, die beispielsweise durch Einfüllen verunreinigten Wassers 52 in das Wasserreservoir in letzteres gelangen können, mit der durch den Strömungskanal 4 in den Innenraum 2 transportierten Luft auf die Proben gelangen, da stromabwärts des Wasserreservoirs im Strömungskanal 4 ein HEPA-Filter 61 vorhanden ist. Dies führt nicht nur zum bestmöglichen Schutz der Proben, sondern verlängert auch die Zeit, bis zu der das Wasser 52 im Wasserreservoir 5 wegen zu großer Verschmutzung oder Verkeimung ausgetauscht werden muss. Damit ist der Wartungsaufwand gegenüber herkömmlichen Inkubatoren erheblich reduziert.

Ein weiterer erheblicher Vorteil des erfindungsgemäßen Inkubators besteht in der guten Temperatur- und Feuchtekonstanz, die im Innenraum 2 eingehalten werden kann. So ermöglicht die räumliche Trennung von Wasserreservoir 5 und Innenraum 2 sowie die getrennte Beheizbarkeit des Wasserreservoirs mittels Heizvorrichtung 51 in Bezug auf die Temperierung des Innenraums 2 durch die Wandheizung 37 eine erheblich bessere und schnellere Regulierung der Innenraumtemperatur. Letztere wird beispielsweise in Abhängigkeit der Messergebnisse geregelt, die vom Temperatursensor 90 im Innenraum erhalten werden. Zur Messung der Feuchtigkeit im Innenraum ist ein weiterer Sensor 91 im Innenraum 2 angeordnet. Zudem sind Sensoren 92 und 93 vorhanden, die anzeigen, dass die Außentür 10 bzw. die Innentür 36 geöffnet wurden. Diese Information erlaubt eine schnelle Reaktion auf Störungen des Systems. Die Öffnung der Türen 10 und 36 hat zur Folge, dass die Temperatur im Innenraum üblicherweise abfällt und die Innenraum-Feuchtigkeit sinkt. Um dieser Störung entgegenzuwirken, ist es sinnvoll, die Wandheizung 37 stärker zu betreiben und vor allen Dingen die Leistung der Heizvorrichtung 51 und damit die Temperatur des Wassers im Wasserreservoir 5 zu erhöhen, um den Feuchtigkeitsverlust im Innenraum möglichst schnell auszugleichen und so ein Austrocknen der Proben zur verhindern. Da erfindungsgemäß in einer bevorzugten Ausführungsform die Heizvorrichtung 51 unabhängig von der Wandheizung 37 betrieben wird, kann die Leistung der Heizvorrichtung 51 im Vergleich zur Wandheizung 37 stärker erhöht werden, so dass ein Feuchtigkeitsverlust erheblich schneller ausgeglichen werden kann als bei gleicher Beheizung. Die Temperatur des Wassers im Wasserreservoir 5 wird dabei mittels eines Temperatursensors 50 gemessen, der hier in einem Seitenwandbereich des Wasserreservoirs angeordnet ist. In einer bevorzugten Ausführungsform befindet sich der Temperatursensor 50 in einer Position, die dem minimal gewünschten Wasserstand im Wasserreservoir 5 entspricht. In dieser Weise kann der Temperatursensor 50 gleichzeitig als Wasserstandsanzeige eingesetzt werden. Sinkt nämlich der Wasserspiegel unter den gewünschten minimalen Wasserstand ab, fällt damit auch der Temperatursensor 50 trocken. Dies äußerst sich in einer sprunghaften Änderung der vom Temperatursensor gemessenen Temperaturen. Stellt eine hier nicht dargestellte Auswerte- und Steuereinheit einen derartigen Temperatursprung am Sensor 50 fest, wird eine Warnung ausgegeben, die den niedrigen Wasserstand signalisiert, und gegebenenfalls wird dafür gesorgt, dass Wasser 52 durch eine Füllvorrichtung automatisch nachgefüllt wird.

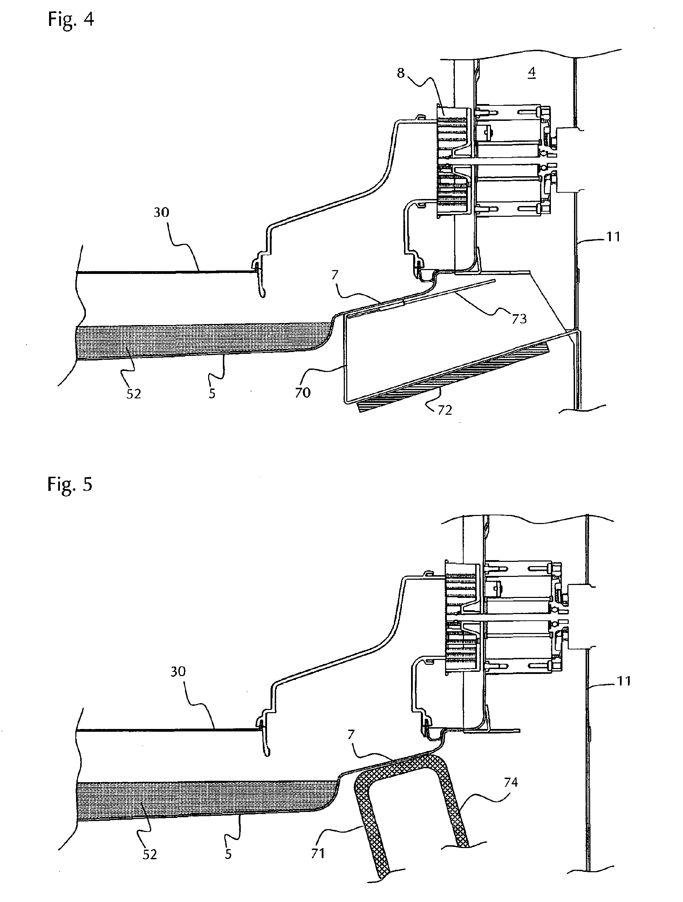

Wird nach einer Störung und der dadurch hervorgerufenen erhöhten Heizleistung der Heizvorrichtung 51 die Ursache der Störung durch Schließen der Türen 36 und 10 wieder behoben, steigen Innenraumtemperatur und Luftfeuchtigkeit im Innenraum sehr rasch wieder auf die gewünschten Sollwerte an. Wegen der relativ starken Aufheizung des Wasserreservoirs 5 besteht allerdings nun die Gefahr, dass es zu einem Übersprung der Temperatur und Luftfeuchtigkeit im Innenraum kommt. Wegen der separaten Regelbarkeit der Wandheizung 37 und der Heizvorrichtung 51 für das Wasserreservoir kann auf zu rasche Temperaturanstiege jedoch erheblich schneller und zielgerichteter reagiert werden, als dies bei einer Verbindung der beiden Heizvorrichtungen der Fall war. Gleiches gilt im Hinblick auf die Einstellung der Luftfeuchtigkeit im Innenraum. Um letztere besonders genau einhalten und möglichst rasch auf den Sollwert einstellen zu können, sieht die Erfindung als zusätzliche Maßnahme die spezielle Anordnung eines Kältepunktes 7 vor. Der Kältepunkt 7 ist ein Bereich reduzierter Temperatur, in dem überschüssige Feuchtigkeit auskondensieren kann. Erfindungsgemäß ist dieser Kältepunkt 7, der bislang im Innenraum 2 meist an einem unteren Bereich einer der Seitenwände angeordnet war, nun wie schon das Wasserreservoir 5 aus dem Innenraum heraus in einen Bereich unter dem Boden 30 verlegt. Konkret befindet sich der Kältepunkt 7 stromabwärts des Wasserreservoirs, mit dessen Endbereich verbunden. Wie sich insbesondere Figur 4 entnehmen lässt, ist der Kältepunkt 7 an einem verlängerten Endabschnitt der Wasserwanne 5 angeordnet. Dieser Endabschnitt liegt oberhalb des Wasserspiegels im Wasserreservoir 5 und ist als schräg abfallende Rampenfläche oder herausgezogener Randabschnitt des Wasserreservoirs ausgebildet. Im Bereich des Kältepunktes 7 auskondensierende Feuchtigkeit kann daher in die Wasserwanne 5 zurückfließen.

Die Temperatur des Kältepunktes 7 kann im Beispiel gezielt eingestellt werden. Hierfür ist erstens eine Temperaturbrücke in Form eines Aluminiumstreifens 70 wärmeleitend mit dem Kältepunkt 7 kontaktiert. Eine gute Wärmeleitfähigkeit im Kontaktbereich zwischen Kältepunkt 7 und Aluminiumstreifen 70 kann beispielsweise durch Auftrag einer Wärmeleitpaste sichergestellt werden. Der Aluminiumstreifen 70 ist so gewinkelt, dass das nicht mit dem Kältepunkt 7 kontaktierte Ende auf die Außenseite des Außengehäuses 11 herausgeführt ist. Der Aluminiumstreifen 70 dient damit als Kältebrücke, der die geringere Außentemperatur der Umgebung des Inkubators auf den Kältepunkt 7 überträgt. Zusätzlich zu dieser passiven Kältebrücke ist zweitens eine aktive Temperiervorrichtung 72 mit dem Aluminiumstreifen 70 verbunden. Bei der Temperiervorrichtung kann es sich beispielsweise um eine elektrische Kühl- und Heizvorrichtung handeln. Mit dieser kann der Kältepunkt 7 gezielt auf eine vorgegebene Temperatur eingestellt werden. So ist es nicht nur möglich, den Kältepunkt abzukühlen, wenn überschüssige Feuchtigkeit aus der im Strömungskanal 4 zirkulierten Luft auskondensiert werden muss, sondern es ist ebenfalls möglich, für bestimmte Anwendungen die Temperatur des Kältepunktes gezielt zu erhöhen, um eine Kondensation gerade zu vermeiden. Um eine Regelung der Temperatur des Kältepunktes 7 in Abhängigkeit von der gewünschten Anwendung möglich zu machen, ist mit dem Aluminiumstreifen 70 weiterhin ein Temperatursensor 73 kontaktiert, der die Temperatur des Kältepunktes misst.

Die Erfassung der Messergebnisse der verschiedenen beschriebenen Sensoren, die Auswertung der Messergebnisse und die auf dieser Auswertung beruhende Steuerung der einzelnen Heizvorrichtungen, der Gebläseleistung usw. erfolgt in an sich bekannter Weise mittels einer üblichen Auswerte- und Steuereinrichtung. Dies ermöglicht in Abhängigkeit von den jeweils ermittelten Temperatur-, Feuchtigkeits- und sonstigen Messwerten eine schnelle und exakte Regelung von Temperatur und Feuchtigkeit im Innenraum des Inkubators und gewährleistet so, kombiniert mit der speziellen Strömungsführung innerhalb des Innenraumes 2 des Inkubators, eine nach Wunsch und in Bezug auf die zu lagernden Proben exakt angepasste Innenraumatmosphäre.

Diese Innenraumatmosphäre kann zudem nicht nur aus feuchter Luft bestehen, sondern es können weitere Gase zugeführt werden. Hierbei kann es sich beispielsweise um Stickstoff oder Kohlendioxid handeln. Die Zufuhr dieser weiteren Gase kann prinzipiell auf jede im Stand der Technik übliche Art und Weise erfolgen. Bevorzugt ist es im Rahmen dieser Erfindung jedoch, diese weiteren Gase ebenfalls in den Strömungskanal 4 zuzuführen und nicht unmittelbar in den Innenraum 2. In letzterem Fall besteht nämlich die Gefahr der Austrocknung der Proben, da die kommerziell verfügbaren Gase üblicherweise getrocknete Gase mit sehr geringer Luftfeuchtigkeit sind. Bevorzugt ist es daher, die weiteren Gase so in den Strömungskanal 4 einzuleiten, dass sie zunächst über das Wasserreservoir 5 geführt werden und sich dort mit Feuchtigkeit anreichern, bevor sie über die Luftausströmöffnung 41 dem Innenraum 2 zugeführt werden. Zu diesem Zweck kann beispielsweise in der nähe der Lufteinströmöffnung 40 für die im Innenraum zirkulierte Luft eine weitere Einströmöffnung 42 vorhanden sein, durch die Gas wie Stickstoff oder Kohlendioxid zugeführt wird. Dieses Gas mischt sich dann im Strömungskanal 4 mit der Luft, reichert sich, wie erwähnt, beim Führen über das Wasserreservoir 5 mit Feuchtigkeit an, passiert den HEPA-Filter 61, dann das Gebläse 8 und gelangt anschließend über den restlichen Strömungskanal 4 und den Filter 62 durch die Luftausströmöffnung 41 in den Innenraum 2.

Um eine möglichst gleichmäßige Strömung der Luft bzw. des Luft-Gas-Gemisches im Innenraum über den gesamten Anwendungszeitraum sicherzustellen, können den verschiedenen Filtern 60, 61 und 62 Strömungswiderstandsmesser zugeordnet sein, die ermöglichen, zu erkennen, inwieweit die Filter durch Verschmutzung verstopft sind und der Luftströmung so einen erhöhten Widerstand entgegenstellen. Im gezeigten Fall ist dem Filter 62 in der Nähe der Luftausströmöffnung 41 ein solcher Strömungswiderstandsmesser 63 zugeordnet. Je mehr der Filter 63 verschmutzt, umso mehr erhöht sich der Strömungswiderstand dieses Filters, und entsprechend steigen die Messwerte, die der Sensor 63 liefert. Als Folge dieser Messwerterhöhung kann die Gerätesteuerung die Gebläseleistung des Gebläses 8 heraufsetzen. Auf diese Weise kann das Volumen der geförderten Luft über die Zeit konstant gehalten werden.

Fig. 5 zeigt schematisch eine alternative Art der Temperierung des Kältepunktes 7. Anstelle des Aluminiumstreifens der Fig. 4 ist hier ein Hohlkörper 71 in Form eines Schlauchsystems vorhanden. Das Schlauchsystem bildet einen Förderkreislauf, innerhalb dessen ein wärmetransportierendes Fluid 74 wie zum Beispiel Wasser gefördert werden kann. Das Fluid kann in einem im Kreislauf befindlichen, hier aber nicht gezeigten Reservoir mittels einer Heiz- und Kühlvorrichtung auf eine gewünschte Temperatur gebracht werden. In einer vereinfachten Variante kann ein Abschnitt des Förderkreislaufs zur Kühlung einfach in eine kältere Umgebung wie das Äußere des Inkubators verlegt werden. Soll der Kältepunkt 7 nicht gekühlt werden - beispielsweise außerhalb des normalen Inkubationsbetriebs wie während eines Desinfektionsvorgangs - wird das Fluid aus dem Hohlkörper 71 abgelassen. Damit unterbleibt die Wärmeabfuhr vom Kältepunkt 7 und dieser gleicht sich der Temperatur in seiner Umgebung an. Soll der Kältepunkt wieder gekühlt werden, wird der Hohlkörper 71 erneut mit Fluid gefüllt.

## Patentansprüche

1. Laboratoriumsbrutschrank (1), insbesondere Begasungsbrutschrank, mit einem eine Tür (10) umfassenden Außengehäuse (11) und einem einen Innenraum (2) umgebenden Innengehäuse (3), das einen Boden (30), eine Decke (31), drei Seitenwände (32, 33, 34) und eine Seitenfläche (35) aufweist, die mit der Tür (10) oder einer zusätzlichen Innentür (36) verschließbar ist, und in welchem ein Strömungskanal (4) vorhanden ist, der wenigstens eine Lufteinströmöffnung (40) in einem Endbereich und wenigstens eine Luftausströmöffnung (41) in einem anderen Endbereich aufweist, wobei die wenigstens eine Luftausströmöffnung (41) so ausgebildet ist, dass ausströmende Luft am Rand des Innenraumes (2) entlang wenigstens einer der Seitenwände (32, 33, 34) und/oder der Seitenfläche (35) geführt wird, und worin innerhalb des Strömungskanals (4) ein beheizbares Wasserreservoir (5) derart angeordnet ist, dass im Strömungskanal fließende Luft über oder durch das Wasserreservoir (5) geleitet wird.

2. Laboratoriumsbrutschrank nach Anspruch 1,
worin der Strömungskanal (4) zwischen Außengehäuse (11) und Innengehäuse (3) angeordnet ist.

3. Laboratoriumsbrutschrank nach Anspruch 1 oder 2,
worin die wenigstens eine Luftausströmöffnung (41) in einem Randbereich der Decke (31) und/oder in einem der Decke (31) benachbarten Randbereich wenigstens einer der Seitenwände (32, 33, 34) angeordnet ist.

4. Laboratoriumsbrutschrank nach einem der Ansprüche 1 bis 3,
worin sich die wenigstens eine Lufteinströmöffnung (40) in einem Außenrandbereich des Bodens (30) und bevorzugt unterhalb der wenigstens einen Luftausströmöffnung (41) befindet.

5. Laboratoriumsbrutschrank nach einem der Ansprüche 1 bis 4,
worin die wenigstens eine Luftausströmöffnung (41) im Randbereich der Decke (31) oberhalb der Tür (10) oder der Innentür (36) und die wenigstens eine Lufteinströmöffnung (40) in einem der Unterkante der Tür (10) oder Innentür (36) benachbarten Bereich angeordnet ist, sodass Luft von oben nach unten entlang der Tür (10) oder Innentür (36) geführt wird.

6. Laboratoriumsbrutschrank nach einem der Ansprüche 1 bis 5,
worin die wenigstens eine Luftausströmöffnung (41) als Schlitzdüse ausgebildet ist.

7. Laboratoriumsbrutschrank nach einem der Ansprüche 1 bis 6,
worin das Wasserreservoir (5) unterhalb des Bodens (30), insbesondere in Form einer nach oben offenen Wasserwanne, angeordnet ist.

8. Laboratoriumsbrutschrank nach einem der Ansprüche 1 bis 7,
worin ein Temperatursensor (50) zur Messung der Wassertemperatur im Wasserreservoir, bevorzugt auf Höhe des minimalen Wasserspiegels, vorgesehen ist.

9. Laboratoriumsbrutschrank nach einem der Ansprüche 1 bis 8,
worin für das Wasserreservoir (5) eine von einer Heizvorrichtung für den Innenraum (2) gesonderte Heizvorrichtung (51) vorgesehen ist, welche bevorzugt in Abhängigkeit von Messergebnissen des Temperatursensors (50) regelbar ist.

10. Laboratoriumsbrutschrank nach einem der Ansprüche 1 bis 9,
worin im Strömungskanal (4) vor und/oder nach dem Wasserreservoir (5) ein Filter (60, 61), bevorzugt ausgewählt aus einer luftdurchlässigen Membran, einem HEPA- oder ULPA-Filter, angeordnet ist.

11. Laboratoriumsbrutschrank nach Anspruch 10,
worin im Strömungskanal (4) in Strömungsrichtung nach dem Wasserreservoir (5) ein Kältepunkt (7) vorgesehen ist.

12. Laboratoriumsbrutschrank nach Anspruch 11,
worin zur Temperierung des Kältepunktes (7) ein Temperiermittel vorgesehen ist, das ausgewählt ist aus
- einer Temperaturbrücke, die mit dem Außengehäuse (11) wärmeleitend kontaktiert ist, bevorzugt in Form eines Metall- und insbesondere Aluminiumstreifens (70),
- einem mit einem Kühlfluid (74) gefüllten, vorzugsweise entleerbaren, Hohlkörper (71),
- einer Temperiervorrichtung (72) zum aktiven Temperieren, bevorzugt ausgewählt aus einer elektrischen Kühl- und/oder Heizvorrichtung und einem Gebläse.

13. Laboratoriumsbrutschrank nach Anspruch 11 oder 12,
worin ein Temperatursensor (73) zur Messung der Temperatur des Kältepunktes (7) vorgesehen ist und worin die Temperiervorrichtung (72) bevorzugt in Abhängigkeit von Messergebnissen des Kältepunkt-Temperatursensors (73) regelbar ist.

14. Laboratoriumsbrutschrank nach einem der Ansprüche 1 bis 13,
welcher wenigstens einen der folgenden Sensoren aufweist:
- einen Sensor (90) zur Bestimmung der Temperatur im Innenraum (2),
- einen Sensor (91) zur Bestimmung der Feuchtigkeit im Innenraum (2),
- einen Sensor (92) zur Bestimmung, ob die Tür (10) geöffnet oder geschlossen ist,
- einen Sensor (93) zur Bestimmung, ob die Innentür (36) geöffnet oder geschlossen ist,
worin die Heizvorrichtung für das Wasserreservoir (5) und/oder die Temperiervorrichtung (72) für den Kältepunkt (7) in Abhängigkeit von Messergebnissen wenigstens eines der Sensoren regelbar ist.

15. Laboratoriumsbrutschrank nach einem der Ansprüche 1 bis 14,
worin im Strömungskanal (4) ein Gebläse (8), insbesondere ein Radialgebläse, vorzugsweise stromabwärts des Wasserreservoirs (5), angeordnet ist.

16. Laboratoriumsbrutschrank nach Anspruch 15,
worin zwischen Gebläse (8) und der wenigstens einen Luftausströmöffnung (41), vorzugsweise am Ende des Strömungskanals (4), ein Filter (62), insbesondere eine HEPA- oder ULPA-Filter, angeordnet ist.

17. Laboratoriumsbrutschrank nach einem der Ansprüche 10 bis 16,
worin dem Filter (62) ein Strömungswiderstandsmesser (63) zugeordnet ist und worin die Leitung des Gebläses (8) in Abhängigkeit von Messergebnissen des Strömungswiderstandsmessers (63) einstellbar ist.

18. Laboratoriumsbrutschrank nach einem der Ansprüche 1 bis 17,
Mittel zum Einleiten von Gas in den Innenraum (2) aufweisend, worin das Gas in den Strömungskanal (4), vorzugsweise stromaufwärts des Wasserreservoirs (5), einleitbar ist.

## Claims

1. A laboratory incubator (1), more particularly a gassing incubator, with an outer housing (11) comprising a door (10) and an inner housing (3) surrounding an internal chamber (2), which inner housing comprises a floor (30), a ceiling (31), three side walls (32, 33, 34) and a lateral surface (35) that can be closed by said door (10) or by an additional inner door (36), and in which a flow channel (4) is provided which comprises at least one air inlet opening (40) in an end region and at least one air outlet opening (41) in a different end region, said at least one air outlet opening (41) being formed such that effluent air is guided along at least one of the side walls (32, 33, 34) and/or the lateral surface (35) in the peripheral region of said internal chamber (2), and a heatable water reservoir (5) being disposed within said flow channel (4) such that air flowing in the flow channel is passed across or through said water reservoir (5).

2. The laboratory incubator according to claim 1,
wherein said flow channel (4) is disposed between said outer housing (11) and said inner housing (3).

3. The laboratory incubator according to claim 1 or claim 2,
wherein said at least one air outlet opening (41) is disposed in a peripheral region of said ceiling (31) and/or in a peripheral region of at least one of said side walls (32, 33, 34) adjacent to said ceiling (31).

4. The laboratory incubator according to any one of claims 1 to 3,
wherein said at least one air inlet opening (40) is situated in an outer peripheral region of said floor (30) and preferably below the at least one air outlet opening (41).

5. The laboratory incubator according to any one of claims 1 to 4,
wherein the at least one air outlet opening (41) is disposed in the peripheral region of said ceiling (31) above said door (10) or said inner door (36) and the at least one air inlet opening (40) is disposed in a region adjacent to the bottom edge of said door (10) or said inner door (36), such that air is guided downwardly along said door (10) or said inner door (36).

6. The laboratory incubator according to any one of claims 1 to 5,
wherein the at least one air outlet opening (41) is implemented as a slot nozzle.

7. The laboratory incubator according to any one of claims 1 to 6,
wherein said water reservoir (5) is disposed beneath said floor (30), more particularly in the form of an upwardly open water trough.

8. The laboratory incubator according to any one of claims 1 to 7,
wherein a temperature sensor (50) for measuring the water temperature in said water reservoir is provided in the water reservoir, preferably at the level of the minimum water level.

9. The laboratory incubator according to any one of claims 1 to 8,
wherein a heating device (51) that is separate from a heating device provided for said internal chamber (2) is provided for said water reservoir (5), which heating device (51) is preferably controllable depending on measurement results of said temperature sensor (50).

10. The laboratory incubator according to any one of claims 1 to 9,
wherein in the flow channel (4) a filter (60, 61) is disposed prior to and/or following the water reservoir (5), which filter is preferably selected from an air-permeable membrane, a HEPA filter or an ULPA filter.

11. The laboratory incubator according to claim 10,
wherein a cold spot (7) is provided in said flow channel (4) in the direction of flow following said water reservoir (5).

12. The laboratory incubator according to claim 11,
wherein for the purpose of adjusting the temperature of said cold spot (7) a temperature control device is provided that is selected from:
- a temperature bridge that is in heat-conducting contact with said outer housing (11) and is preferably in the form of a metal strip, especially an aluminum strip (70),
- a hollow member (71) which is filled with a cooling fluid (74) and is preferably capable of being emptied,
- a temperature control device (72) for effecting active temperature control, preferably selected from an electrical cooling and/or heating device and a fan.

13. The laboratory incubator according to claim 11 or claim 12,
wherein a temperature sensor (73) is provided for the purpose of measuring the temperature of said cold spot (7) and the temperature control device (72) is preferably controllable depending on measurement results of said cold spot temperature sensor (73).

14. The laboratory incubator according to any one of claims 1 to 13,
which comprises at least one of the following sensors:
- a sensor (90) for ascertaining the temperature in the internal chamber (2),
- a sensor (91) for ascertaining the moisture in the internal chamber (2),
- a sensor (92) for ascertaining whether the door (10) is open or closed,
- a sensor (93) for ascertaining whether the inner door (36) is open or closed,
wherein said heating device for said water reservoir (5) and/or said temperature control device (72) for said cold spot (7) is controllable depending on measurement results of at least one of said sensors.

15. The laboratory incubator according to any one of claims 1 to 14,
wherein a fan (8), more particularly a radial fan, is disposed in said flow channel (4), preferably downstream of said water reservoir (5).

16. The laboratory incubator according to claim 15,
wherein a filter (62), more particularly a HEPA filter or an ULPA filter, is disposed between said fan (8) and said at least one air outlet opening (41), preferably at the end of said flow channel (4).

17. The laboratory incubator according to any one of claims 10 to 16,
wherein a flow resistance meter (63) is allocated to the filter (62) and the output of the fan (8) is adjustable according to measurement results of said flow resistance meter (63).

18. The laboratory incubator according to any one of claims 1 to 17,
comprising means for the introduction of gas into the internal chamber (2), wherein said gas can be introduced into said flow channel (4), preferably upstream of said water reservoir (5).

## Revendications

1. Incubateur de laboratoire (1), et plus particulièrement incubateur sous atmosphère gazeuse, avec une enveloppe extérieure (11) comprenant une porte (10) et une enveloppe intérieure (3) entourant une chambre interne (2), ladite enveloppe intérieure comprenant un plancher (30), un plafond (31), trois parois latérales (32, 33, 34) et une surface latérale (35) qui peut être fermée par ladite porte (10) ou par une porte intérieure supplémentaire (36), et où un canal d'écoulement (4) est prévu qui comprend au moins une ouverture (40) d'admission d'air dans une zone d'extrémité et au moins une ouverture (41) d'évacuation d'air dans une zone d'extrémité différente, ladite au moins une ouverture (41) d'évacuation d'air étant conformée de telle sorte que l'air de purge soit guidé le long d'au moins l'une des parois latérales (32, 33, 34) et/ou de la surface latérale (35) dans la zone périphérique de ladite chambre interne (2), et un réservoir d'eau (5) pouvant être chauffé étant disposé dans ledit canal d'écoulement (4) de telle sorte que l'air s'écoulant dans le canal d'écoulement soit envoyé vers ou à travers ledit réservoir d'eau (5).

2. Incubateur de laboratoire selon la revendication 1,
dans lequel ledit canal d'écoulement (4) est disposé entre ladite enveloppe extérieure (11) et ladite enveloppe intérieure (3).

3. Incubateur de laboratoire selon la revendication 1 ou 2,
dans lequel ladite au moins une ouverture (41) d'évacuation d'air est disposée dans une zone périphérique dudit plafond (31) et/ou dans une zone périphérique d'au moins l'une desdites parois (32, 33, 34) adjacente audit plafond (31).

4. Incubateur de laboratoire selon l'une quelconque des revendications 1 à 3,
dans lequel ladite au moins une ouverture (40) d'admission d'air est située dans une zone périphérique extérieure dudit plancher (30), et, de manière préférée, au-dessous de ladite au moins une ouverture (41) d'évacuation d'air.

5. Incubateur de laboratoire selon l'une quelconque des revendications 1 à 4,
dans lequel ladite au moins une ouverture (41) d'évacuation d'air est disposée dans la zone périphérique dudit plafond (31) au-dessus de ladite porte (10) ou de ladite porte intérieure (36), et ladite au moins une ouverture (40) d'admission d'air est disposée dans une zone adjacente au rebord inférieur de ladite porte (10) ou de ladite porte intérieure (36), de telle sorte que l'air soit guidé vers le bas le long de ladite porte (10) ou de ladite porte intérieure (36).

6. Incubateur de laboratoire selon l'une quelconque des revendications 1 à 5,
dans lequel ladite au moins une ouverture (41) d'évacuation d'air est conformée en buse plate.

7. Incubateur de laboratoire selon l'une quelconque des revendications 1 à 6,
dans lequel ledit réservoir d'eau (5) est disposé sous ledit plancher (30), et plus particulièrement en prenant la forme d'un bac ouvert sur le haut.

8. Incubateur de laboratoire selon l'une quelconque des revendications 1 à 7,
dans lequel un capteur de température (50) pour mesurer la température de l'eau dans ledit réservoir d'eau est présent dans le réservoir d'eau, de manière préférée au niveau de la hauteur d'eau minimale.

9. Incubateur de laboratoire selon l'une quelconque des revendications 1 à 8,
dans lequel un dispositif de chauffage (51), qui est distinct d'un dispositif de chauffage prévu pour ladite chambre interne (2), est prévu pour ledit réservoir d'eau (5), ledit dispositif de chauffage (51) étant, de manière préférée, contrôlable en fonction des résultats de mesures dudit capteur de température (50).

10. Incubateur de laboratoire selon l'une quelconque des revendications 1 à 9,
dans lequel, dans le canal d'écoulement (4) est disposé un filtre (60, 61) avant et/ou juste après le réservoir d'eau (5), ledit filtre étant de manière préférée choisi entre une membrane perméable à l'air, un filtre HEPA ou un filtre ULPA.

11. Incubateur de laboratoire selon la revendication 10,
dans lequel un point froid (7) est prévu dans ledit canal d'écoulement (4) dans la direction de l'écoulement après ledit réservoir d'eau (5).

12. Incubateur de laboratoire selon la revendication 11,
dans lequel, pour régler la température dudit point froid (7), un dispositif de régulation de la température est prévu, qui est choisi parmi:
- un pont thermique qui est en contact thermo-conducteur avec ladite enveloppe extérieure (11) et se présente de préférence sous la forme d'une bande de métal, en particulier une bande d'aluminium (70),
- un élément creux (71) qui est rempli d'un fluide réfrigérant (74) et peut, de manière préférée, être vidé,
- un dispositif (72) de régulation de la température destiné à effectuer une régulation active de la température, de manière préférée choisi entre un dispositif électrique refroidisseur et/ou chauffant et un ventilateur.

13. Incubateur de laboratoire selon la revendication 11 ou la revendication 12,
dans lequel un capteur de température (73) est prévu pour mesurer la température dudit point froid (7) et le dispositif (72) de régulation de la température est, de manière préférée, contrôlable en fonction des résultats de mesures dudit capteur de température (73) du point froid.

14. Incubateur de laboratoire selon l'une quelconque des revendications 1 à 13,
qui comprend au moins l'un des capteurs suivants :
- un capteur (90) pour déterminer la température dans la chambre interne (2),
- un capteur (91) pour déterminer l'humidité dans la chambre interne (2),
- un capteur (92) pour déterminer si la porte (10) est ouverte ou fermée,
- un capteur (93) pour déterminer si la porte intérieure (36) est ouverte ou fermée, dans lequel ledit dispositif de chauffage pour ledit réservoir d'eau (5) et/ou ledit dispositif (72) de régulation de la température pour ledit point froid (7) peu(ven)t être contrôlé(s) en fonction des résultats de mesures d'au moins l'un desdits capteurs.

15. Incubateur de laboratoire selon l'une quelconque des revendications 1 à 14,
dans lequel un ventilateur (8), et plus particulièrement un ventilateur radial, est disposé dans ledit canal d'écoulement (4), de manière préférée en aval dudit réservoir d'eau (5).

16. Incubateur de laboratoire selon la revendication 15,
dans lequel un filtre (62), et plus particulièrement un filtre HEPA ou un filtre ULPA, est disposé entre ledit ventilateur (8) et ladite au moins une ouverture (41) d'évacuation d'air, de manière préférée à l'extrémité dudit canal d'écoulement (4).

17. Incubateur de laboratoire selon l'une quelconque des revendications 10 à 16,
dans lequel un dispositif (63) de mesure de la résistance à l'écoulement est affecté au filtre (62) et la puissance du ventilateur (8) est réglable en fonction des résultats de mesures du dispositif (63) de mesure de la résistance à l'écoulement.

18. Incubateur de laboratoire selon l'une quelconque des revendications 1 à 17, comprenant des moyens pour l'introduction du gaz dans la chambre interne (2), par lesquels ledit gaz peut être introduit dans ledit canal d'écoulement (4), de manière préférée en amont dudit réservoir d'eau (5).
